# EUROPÄISCHE PATENTSCHRIFT

(11) **EP 2 726 126 B1**
(45) Veröffentlichungstag und Bekanntmachung des Hinweises auf die Patenterteilung: **12.04.2017**
(21) Anmeldenummer: 12702977.5
(22) Anmeldetag: 03.02.2012
(51) Int. Cl.: A61M 16/01, A61M 16/08, A61M 16/20, A61M 16/22, A61M 16/00, A61M 16/10, A61M 16/18

(54) **BEATMUNGSKREISLAUF MIT MITTELN ZUR STEUERUNG DES ENDEXSPIRATORISCHEN DRUCKES**
RESPIRATORY SYSTEM HAVING MEANS FOR CONTROLLING THE END-EXPIRATORY PRESSURE
CIRCUIT RESPIRATOIRE AYANT DES MOYENS POUR COMMANDER LA PRESSION EXPIRATOIRE FINALE

(30) Priorität: 02.07.2011 DE 102011106406
(43) Veröffentlichungstag der Anmeldung: 07.05.2014
(73) Patentinhaber: Drägerwerk AG & Co. KGaA, 23558 Lübeck (DE)
(72) Erfinder: RAHLF, Till, 23617 Stockelsdorf (DE); HEESCH, Ralf, 23568 Lübeck (DE)
(86) Internationale Anmeldenummer: PCT/EP2012/000495
(87) Internationale Veröffentlichungsnummer: WO 2013/004317

(56) Entgegenhaltungen:
- WO-A1-2010/130290
- GB-A- 2 417 206

## Beschreibung

Die vorliegende Erfindung betrifft ein Anästhesie-oder Beatmungsgerät mit Steuerung eines endexspiratorischen Druckes am Ende der Exspirationsphase eines Patienten, wobei das Anästhesiegerät oder Beatmungsgerät einen Beatmungskreislauf aufweist, in dem ein exspiratorischer Drucksensor, ein inspiratorischer Drucksensor, ein inspiratorischer Durchflusssensor, ein exspiratorischer Durchflusssensor, ein steuerbares exspiratorisches Ventil und ein Beatmungsantrieb angeordnet sind, und einer Betriebs- und Ansteuerungseinheit, die mit den vorhergenannten Komponenten in dem Beatmungskreislauf verbunden ist.

Bei.der maschinellen Beatmung eines Patienten durch ein Anästhesie- oder Beatmungsgerät wird am Ende der Ausatmung der Druck so weit vermindert, dass der Patient ausatmen kann. Dabei wird das Druckniveau nicht vollständig auf den herrschenden Umgebungsdruck abgesenkt, sondern es verbleibt ein Restdruck, der sogenannte positiv-endexspiratorische Druck (PEEP), in der Lunge. Dieser Druck wird mittels des Anästhesie- oder Beatmungsgerätes während der Ausatmung bis zur nächsten Einatmung konstant gehalten. Dabei werden durch einen Druckregler im Anästhesie- oder im Beatmungsgerät etwaige Leckagen oder Undichtigkeiten in der Verbindung vom Anästhesie- oder Beatmungsgerät bis zum Patienten ausgeglichen, indem der Druckregler soweit ein Volumen mittels einer Durchflussmengengabe nachführt, dass der positiv-endexspiratorische Druck in der Lunge des Patienten gehalten werden kann. Durch diesen leichten Überdruck in der Lunge gegenüber dem Umgebungsdruck wird durch eine Dehnung der Lunge sichergestellt, dass die geöffneten Areale in der Lunge nicht während der Ausatmung in der Zeit bis zur nächsten Einatmung wieder zusammenfallen und dadurch die Austauschfläche der Lunge nicht verringert wird.

In Anästhesiekreisläufen wird direkt am Anschlussstück zum Patienten, in dem der exspiratorische und der inspiratorische Beatmungsschlauch zusammengeführt werden, dem sogenannten Y-Stück, in vielen Fällen ein absaugendes Messsystem mittels eines Schlauches angeschlossen, wobei mit einem AbsaugeVolumenstrom Gas direkt vom Patienten in das Messsystem geführt wird, in der analysiert wird, wie die physiologischen Beatmungsparameter des Patienten sind. Dabei werden beispielsweise die Sauerstoffkonzentration und die Kohlendioxid-Konzentration (C_{CO₂}) über der Zeit aufgezeichnet. Von diagnostischer und therapeutischer Bedeutung ist dabei insbesondere die Kohlendioxid-Konzentration (C_{CO₂}) am Ende der Ausatmung des Patienten, die sogenannte endtidale Konzentration (etCO₂). Dies lässt einen Rückschluss darauf zu, inwieweit die Beatmung des Patienten, d. h. die Versorgung mit Sauerstoff ausreichend ist. Auf Basis dieser Sauerstoff- und Kohlendioxid-Konzentrationswerte (C_{CO₂}) entscheidet der Arzt über Anpassungen der Beatmung, beispielsweise der Beatmungsfrequenz, des Minutenvolumens, der Druckeinstellungen, sowie der gewählten, verabreichten Sauerstoffkonzentration.

Die Nachführung des positiv-endexspiratorischen Druckes durch einen Druckregler im Beatmungskreislauf bewirkt, dass direkt zum Patienten an das sogenannte Y-Stück frisches Gas geliefert wird. Dieses frische Gas vermischt sich dort mit der Ausatemluft des Patienten. Vom Patienten, vom Y-Stück, wird diese vermischte Luft zur Messanordnung hin abgesaugt. Die Absaugung erfolgt typischerweise mit einer Absaugeleitung, typischerweise mittels eines sehr dünnen Absaugeschlauchs mit einem Innen- Durchmesser im Bereich von 0,5 mm bis 1,5 mm über eine Länge von 1,5 - 3,0 m. Ein Absaugevolumenstrom von typischerweise ungefähr 0,2 l/min befördert die Luft durch den Absaugeschlauch vom Patienten in die Messanordnung. Damit gelangt das Gas vom Y- Stück bedingt durch den Weg und die Art der Absaugung mit einer Verzögerung im Bereich von ungefähr 0,8 bis zu 3,5 Sekunden zur Messanordnung. Sind in der Absaugeleitung auf dem Weg zur Messanordnung weitere Komponenten mit einem zusätzlichem Volumen, wie beispielsweise eine Wasserfalle, angeordnet und berücksichtigt man das Volumen in der Messanordnung selbst, sowie die erforderliche Messzeit zur Bestimmung der Kohlendioxid- Konzentration in der Messanordnung , so erhöht sich die Verzögerung zwischen dem Ort der Messprobe am Patienten bis zu einem messtechnisch erfassten und angezeigten Wert einer Kohlendioxid- Konzentration insgesamt auf Wert im Bereich von ungefähr 3 Sekunden bis zu 10 Sekunden.
Eine solche Verzögerung entspricht ungefähr einer Anzahl von weniger als einem Atemzyklen bis hin zu 2 Atemzyklen für einen Erwachsenen, von 1 bis 6

Atemzyklen für ein Kleinkind, sowie von 3 bis 10 Atemzyklen für ein Neugeborenes.

Abhängig von der Art der Druckregelung und der Situation am Patienten, am Atemsystem, den gewählten Beatmungsparametern und den im System vorhandenen Leckagen führt die geregelte Nachführung des positiv-endexspiratorischen Druckes dazu, dass die Kohlendioxid-Konzentration (C_{CO₂}) an der Messanordnung nicht der Konzentration entspricht, die im Rachenraum und Bronchialraum des Patienten bei der Ausatmung vorliegt. Ein Atemkreislauf mit einem Regler zur Einstellung eines positiv-endexspiratorischen Druckes ist aus der Schrift US 4,082,093 bekannt.

Es ist die Aufgabe der vorliegenden Erfindung, ein Beatmungs- oder Anästhesiegerät anzugeben, das es ermöglicht, den positiv-endexspiratorischen Druck in einem Atemkreislauf eines Beatmungs- oder Anästhesiegerätes in der Art einzustellen, dass sich keine Effekte durch die Druckregelung in den Messwerten der Kohlendioxid-Konzentration (C_{CO₂}) in der CO₂-Messanordnung ergeben.

Die Aufgabe wird gelöst durch ein Beatmungs- oder Anästhesiegerät mit den Merkmalen des Patentanspruchs 1 gelöst.

Vorteilhafte Ausführungsformen ergeben sich aus den abhängigen Ansprüchen.

Es ist in erfindungsgemäßer Weise vorgesehen, dass die Betriebs- und Ansteuerungseinheit dazu eingerichtet ist, das steuerbare exspiratorische Ventil und den Beatmungsantrieb in ihrem Betrieb so zu regeln, dass der Druckverlauf während der Exspirationsphase vom Ende der Inspirationsphase bis zum Beginn der nächsten, folgenden Inspirationsphase von einem ersten oberen Druckwert zu einem ersten unteren Druckwert einen fallenden Verlauf mit höchstens stückweise konstanten Teilintervallen aufweist. Dadurch wird der positiv-endexspiratorische Druck im Beatmungskreislauf des Anästhesie- oder Beatmungsgerätes in der Weise gesteuert, dass sich die Vermischung am Y-Stück nicht auf die Messwerte der dort ansetzenden, absaugenden Gaskonzentrationsmessung, insbesondere der CO₂-Messun auswirkt. Die Steuerung des Druckreglers erfolgt dabei in der Weise, dass der positiv-endexspiratorische Druck für die Ausatemphase, - die sogenannte Exspirationsphase -, des Patienten, nicht auf einem absolutkonstanten Druckniveau gehalten wird, sondern dass das Druckniveau des positiv-endexspiratorischen Drucks (PEEP) am Ende der Exspirationsphase gegenüber dem Druckniveau des positiv-endexspiratorischen Drucks (PEEP) am Beginn der Exspirationsphase geringer gewählt und ausgeregelt wird. Dazu wird erfindungsgemäß im Verlauf der Exspirationsphase anstatt eines konstanten Druckniveaus eine fallende Rampe als Stellwert für den Druckregler des positiv-endexspiratorischen Druckes (PEEP) zur Regelung verwendet, die höchstens stückweise konstante Teiliritervalle umfasst. Die Druckniveaus am Ende und am Anfang der Exspirationsphase werden dabei vorzugsweise aus einem vorbestimmten mittleren Wert des positiv-endexspiratorischen Drucks (PEEP) ermittelt. In der Einatemphase, - der sogenannten Inspirationsphase -, des Patienten findet keine erfindungsgemäße Beeinflussung der Druckregelung statt.

Die fallende Druckrampe kann durch einen ersten oberen Druckwert und einen ersten unteren Druckwert beschrieben werden. Der Mittelwert zwischen dem ersten oberen und dem ersten unteren Druckwert stellt den gewünschten positiv-endexspiratorischen Druck dar, so dass sich für den Patienten im anliegenden Druckverhältnis zum Umgebungsdruck im Mittel während der Dauer der Ausatemphase keinerlei Änderung gegenüber einem konstant anliegenden, auf einem gleichbleibenden Druckniveau ausgeregelten positiv-endexspiratorischen Druck ergibt.

Die Dauer der Ausatemphase und der Einatemphase wird durch die gewählten Beatmungsparameter wie die Beatmungsfrequenz in Kombination mit dem sogenannten I/E-Verhältnis, d. h. dem Verhältnis zwischen Inspirationsphase und Exspirationsphase definiert. Aus dieser Dauer der Exspirationsphase und dem gewünschten, vom Anwender eingestellten positiv-endexspiratorischen Druckwert werden der erste obere Druckwert und der erste untere Druckwert ermittelt.

Soweit im Folgenden die Art und Weise der Regelung des Druckverlaufs beschrieben wird, ist dies so zu verstehen, dass die Betriebs- und Ansteuerungseinheit dazu eingerichtet ist, das steuerbare exspiratorische Ventil und den Beatmungsantrieb so zu steuern, dass der beschriebene Druckverlauf realisiert wird.

In einer bevorzugten Ausführungsform wird die Ermittlung des ersten oberen Druckwertes und des ersten unteren Druckwertes durch einen linear fallenden Verlauf oder durch einen nicht-linear fallenden Verlauf des Druckes in der Exspirationsphase abgebildet.

Unter einem linearen Verlauf wird im Sinne der vorliegenden Erfindung jeglicher Verlauf mit umfasst, der einer linearen Geradengleichung folgt. Unter einem nichtlinearen Verlauf wird im Sinne der vorliegenden Erfindung jeder Verlauf mit umfasst, der einer quadratischen, kubischen Funktion oder einem Polynom höherer Ordnung folgt. Weiterhin sind auch logarithmische oder exponentielle Verläufe mit umfasst so wie in allgemeiner Weise progressiv oder degressiv fallende Funktionsverläufe, die geeignet sind, einen ersten oberen Druckwert und einen ersten unteren Druckwert aus der Dauer der Exspirationsphase und dem mittleren, gewünschten Wert für den positiv-endexspiratorischen Druck zu ermitteln.

In einer weiter bevorzugten Ausführungsform ist der Funktionsverlauf stetig oder unstetig mit fallenden und konstanten Teilintervallen ausgebildet. Letzteres bedeutet, dass eine linear oder eine nicht-linear fallende Funktion keinen in stetiger Weise einen kontinuierlichen Verlauf mit fallender Steigung aufweist, sondern dass der Verlauf gestuft vorgenommen wird. In dieser Variante wird der positiv-endexspiratorischen Druck von einem ersten oberen Druckniveau in Stufen auf ein erstes unteres Druckniveau abgesenkt, wobei sich als mittlerer Wert zwischen dem ersten oberen Druckniveau und dem ersten unteren Druckniveau wieder das mittlere Niveau des positiv-endexspiratorischen Druckes einstellt, das vom Anwender für diesen Patienten gewählt wurde.

In einer weiteren bevorzugten Ausführungsform der vorliegenden Erfindung findet der fallende Verlauf von einem ersten oberen Druckniveau zu einem ersten unteren Druckniveau nicht über die gesamte Dauer der Exspirationsphase statt. In dieser weiteren bevorzugten Ausführungsform ist der Verlauf des positiv-endexspiratorischen Drucks teilweise konstant ausgebildet und teilweise fallend ausgebildet. Dabei wird in dieser weiteren bevorzugten Ausführungsform am Ende der Inspirationsphase der positiv-endexspiratorische Druck bei Beginn der Exspirationsphase auf einem oberen Druckniveau für eine gewisse Dauer gehalten. Ab einem gewissen Zeitpunkt in der Exspirationsphase wird eine Absenkung vom oberen Druckniveau nach einem fallenden Verlauf auf ein unteres Druckniveau vorgenommen. Dadurch ergibt sich eine Kombination aus einem oberen konstanten Druckniveau für eine Verzugszeit vorbestimmter Dauer am Beginn der Exspirationsphase mit einem anschließenden fallenden Druck, so dass sich für den verbleibenden zeitlichen Rest der Exspirationsphase ein stärkerer Gradient vom oberen Druckniveau zum unteren Druckniveau ergibt. In dieser weiteren bevorzugten erfindungsgemäßen Ausführungsform bewirkt dieser verstärkte Druck-Gradient, dass für den Patienten die Phase verlängert wird, aus der CO₂-haltige Luft aus seiner Lunge in seinen Mundraum an das Y-Stück gefördert wird. Diese Variante ist besonders dann vorteilhaft, wenn die Exspirationsdauern vergleichsweise lang sind, d. h. bei geringen Atemfrequenzen von 6 - 10 Atemzügen je Minute ist eine solche Steuerung nach einem fallenden Druckverlauf mit einem für die Verzugszeit vorgeschalteten konstanten Anteil besonders vorteilhaft.

In einer weiteren bevorzugten Ausführungsform werden die Einstellungen am Anästhesie- und Beatmungsgerät, die der Anwender für die Beatmungssteuerung des Patienten vorgenommen hat, nämlich den vorgegebenen PEEP-Einstellungen selbst, vorzugsweise in Form eines vorgegebenen mittleren PEEP-Wertes (P̅E̅E̅P̅), sowie dem Verhältnis zwischen der Einatemzeitdauer und der Ausatemzeitdauer, dem sogenannten I/E-Verhältnis und der Beatmungsfrequenz RR (Respiratory Rate) für die Gestaltung des Verlaufs des Drucks in der Exspirationsphase mit berücksichtigt.

Es können die Eigenschaften der Messvorrichtung mit berücksichtigt werden. Dabei werden sowohl die Länge des Schlauches, der Durchmesser des Schlauches als auch der Absaugevolumenstrom mit berücksichtigt. Aus der Länge des Schlauches und dem Durchmesser des Schlauches ergibt sich das Volumen an Gas, was im Schlauch vom Patienten, nämlich dem Y-Stück, bis zur Messvorrichtung als Gasmenge verzögert in die Messeinrichtung gelangt. Aus dem Absaugevolumenstrom und dem Volumen ergibt sich die Verzögerung, die vom Y-Stück und dem dort anstehenden Druck- und Konzeritrationswert vergeht, bis diese Luftmenge zur Analyse in der Messanordnung angekommen ist. In dieser bevorzugten Ausführungsform wird diese Absaugeverzugszeit dadurch berücksichtigt, dass die Wahl der Verzugszeit am Beginn der Exspirationsdauer und auch die Wahl des oberen Druckwertes und des unteren Druckwertes auf die Messverzögerung für die CO₂-Messung abgestimmt wird.

IEs kann die in der Messanordnung erforderliche Messzeit für die Bestimmung und Auswahl der Verzugszeit am Beginn der Exspirationsdauer und des oberen sowie des unteren Druckwertes mit berücksichtigt werden.

Im Folgenden werden für drei unterschiedliche Patiententypen typische Einstellparameter für Beatmungsfrequenz, positiv-endexspiratorischen Druck und I/E-Verhältnis typische Werte für einen oberen Druckwert und einen unteren Druckwert für einen linear fallenden Verlauf des Drucks in der Exspirationsphase angegeben. Als mittlerer positiv-endexspiratorischer Druck wird ein typischer Wert für die gewählten drei Patiententypen angesetzt. Die drei Patiententypen unterscheiden sich im Wesentlichen dadurch, dass die Beatmung nach unterschiedlichen Kriterien und Einstellparametern erfolgen muss, weil die Lungen der Patienten unterschiedlich den pneumatischen Eigenschaften sind. Dazu zählen Volumen, Dehnbarkeit (Compliance) und pneumatischer Widerstand

(Resistance). Die pneumatischen Eigenschaften von Patienten können verschiedenen Patiententypen in vereinfachter Weise anhand des Körpergewichtes zugeordnet werden.

Zu diesen drei Patiententypen zählen als ein erstes Beispiel ein Erwachsener mit einem typischen Körpergewicht von ungefähr 70 kg, als ein zweites Beispiel wird ein Kind mit einem typischen Körpergewicht von ungefähr 10 kg gewählt, als ein drittes Beispiel wird ein neugeborenes Kind mit einem typischen Körpergewicht von ungefähr 2 kg Körpergewicht gewählt.
Für diese drei Patiententypen ergeben sich jeweils unterschiedliche Werte für das Lungenvolumen, unterschiedliche Atemfrequenzen und unterschiedliche Bedingungen daraus für die Beatmung.
Die nachfolgende Tabelle 1 gibt typische mittlere Werte für Körpergewicht, Lungenvolumen V, Minutenvolumen MV, Beatmungsfrequenz RR, I/E-Verhältnis, Inspirationszeitdauer Tᵢ, Exspirationszeitdauer Tₑ und Inspirationsdruck Pᵢₙₛₚ, sowie Exspirationsdruck (PEEP) an.

| Gewicht | V [mL] | MV [L] | RR [1/min] | I/E-Ratio | Pᵢₙₛₚ [hPa] | PEEP [hPa] | Tᵢ [sec] | Tₑ [sec] |
|---|---|---|---|---|---|---|---|---|
| ∼ 70 kg | 600 | 4,8 | 8 | 1 : 2 | 15 | 5 | 2,5 | 5 |
| ∼ 10 kg | 200 | 3,0 | 15 | 1 : 2 | 15 | 5 | 1,33 | 2,66 |
| ∼ 2 kg | 30 | 1,5 | 50 | 1 : 1 | 15 | 5 | 0,6 | 0,6 |

Aus den Werten dieser Tabelle lassen sich dann unter der Annahme eines linearen Verlaufs in diesem Berechnungsbeispiel ein erster oberer Druckwert und ein erster unterer Druckwert ermitteln.

Die folgende Tabelle 2 zeigt typische mittlere Werte für einen oberen Druckwert (PEEP_{High}) und einen unteren Druckwert (PEEP_{Low}) für die drei Patiententypen Erwachsener, Kind und neugeborenes Kind.

| Gewicht | PEEP [hPa] | PEEP_{High} [hPa] | PEEP_{Low} [hPa] |
|---|---|---|---|
| ∼ 70 kg | 5 | 5,2 | 4,8 |
| ∼ 10 kg | 5 | 5,2 | 4,8 |
| ∼ 2 kg | 5 | 3,2 | 2,8 |

Für die besondere Ausführungsform mit einer Verzugszeit am Beginn der Exspirationsphase, bevor die fallende Druckrampe startet, ist hier für das erste Beispiel mit einem Erwachsenen von 70 kg, gemäß den Tabellen 1 und 2, eine exemplarische Berechnung vorgenommen worden.

Für einen Erwachsenen von 70 Kg ergeben sich mit den Daten als Randbedingungen gemäß der Tabellen 1 und 2 und bei einer gewählten Verzugszeit von der Hälfte der Exspirationsdauer von 5 Sekunden eine Verzugszeit von 2,5 Sekunden ein erster oberer Druckwert von 5,2 hPa und ein erster unterer Druckwert von 4,8 hPa für die Regelung des positiv-endexspiratorischen Drucks in der Ausatemphase eines Patienten.

Durch die Verwendung der Verzugszeit am Beginn der Exspirationsphase wird der Druckgradient gegen Ende der Exspirationsphase steiler gegenüber der Ausführung mit einer bereits zu Beginn der Exspirationsphase fallenden Druckrampe. Der steilere Druckgradient bewirkt, dass der Patient in der Lage ist, nahezu bis zum Ende der Exspiration auszuatmen, so dass die Messung der Kohlendioxid- Konzentration auch noch gegen Ende der Exspiration ohne eine Vermischung durch Inspirationsgas erfolgen kann.

Die Festlegung des oberen und des unteren Druckwertes kann für die besondere Ausführungsform mit einer Verzugszeit am Beginn der Exspirationsphase vor dem Start der fallenden Druckrampe in gleicher Weise wie bei der Verwendung der fallenden Druckrampe ohne eine Verzugszeit erfolgen, so dass sich durch die Verzugszeit, -wie zuvor beschrieben-, lediglich ein steilerer Druckgradient ergibt.

Es ist aber auch in einer technischen Realisierung möglich und im Sinne der vorliegenden Erfindung mit umfasst, den unteren Druckwert gegen Ende der Exspirationsphase weiter abzusenken, so dass sich neben dem steileren Druckgradienten zusätzlich ein größerer Druckunterschied ergibt.
In einer solchen Variante ergeben sich dann beispielsweise nach dem obigen Beispiel für einen Patienten von 70 kg Körpergewicht bei einer gewählten Verzugszeit von der Hälfte der Exspirationsdauer von 5 Sekunden eine Verzugszeit von 2,5 Sekunden ein erster oberer Druckwert von 5,2 hPa bis 5,3 hPa und ein erster unterer Druckwert von ungefähr 4,6 hPa bis 4,7 hPa.

Als ein Kriterium zur Festlegung des oberen und unteren Druckwertes kann angesetzt werden, dass sich der positiv-endexspiratorische Druck im Mittel über die Exspirationsphase für den Patienten nicht von dem positiv-endexspiratorischen Druck bei einer konstanten Ausregelung unterscheidet.

Die vorliegende Erfindung wird nun durch eine Anzahl von Figuren und zugehörigen Figurenbeschreibungen näher erläutert. Es zeigen:
- die Fig. 1: eine Darstellung eines Beatmungskreislaufs,
- die Fig. 2: einen ersten Verlauf des Druckes, des Durchflusses und der Kohlendioxid-Konzentration (C_{CO₂}) über eine Anzahl von zwei Atemzyklen eines Patienten ohne eine Nachführung des positivendexspiratorischen Druckes,
- die Fig. 3: einen zweiten Verlauf des Druckes, des Durchflusses und der Kohlendioxid-Konzentration (C_{CO₂}) über eine Anzahl von zwei Atemzyklen eines Patienten mit einer konstanten Nachführung des positiv-endexspiratorischen Druckes,
- die Fig. 4: einen dritten Verlauf des Druckes, des Durchflusses und der Kohlendioxid-Konzentration (C_{CO₂}) über eine Anzahl von zwei Atemzyklen eines Patienten mit einer Nachführung des positivendexspiratorischen Druckes gemäß einer abfallenden Druckkurve,
- die Fig. 5: eine Variante des Verlaufs nach Fig. 4.
und die

Figuren 6a - 6d Darstellungen im Detail nach den Figuren 4 oder 5.

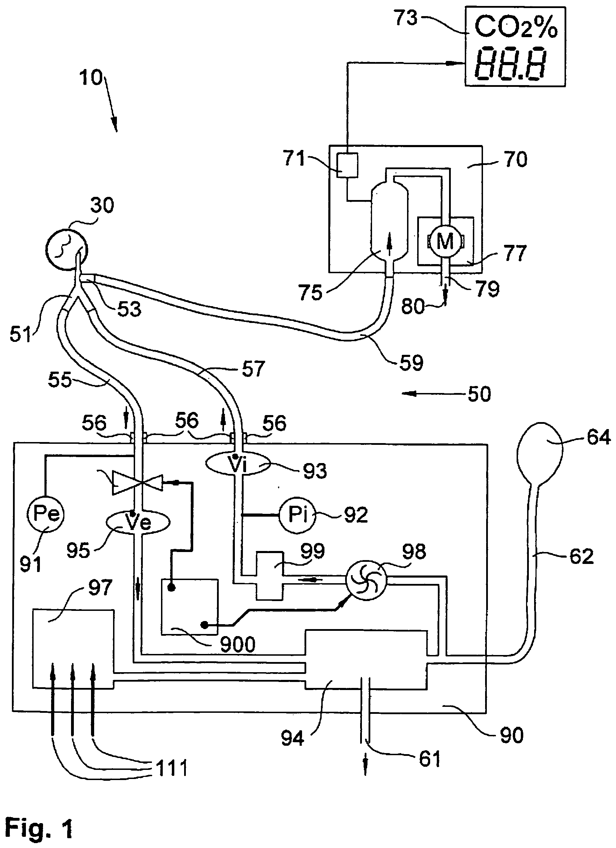

In der Fig. 1 ist schematisch eine Anordnung 10 aus einem Beatmungskreislauf 50 mit einem Patienten 30 und einem Anästhesiegerät 90 sowie einer Messanordnung 70 dargestellt. Die Messanordnung 70 besteht im Inneren aus einer Messeinheit 75, einer Auswerte- und Betriebseinheit 71 sowie einem zugehörigen Anzeigeelement 73. Weiterhin ist in der Messanordnung 70 eine Pumpe 77 angeordnet, welche eine Luftmenge vom Patienten 30 über das Y-Stück 51 und den Absaugeanschluss 53 mittels einer Absaugeleitung 59 in die Messanordnung 70 saugt und an einem Ausgang 79 wieder an die Umgebung 80 bzw. in eine in dieser Figur 1 nicht gezeigte, dafür vorgesehene Fortleitungseinrichtung weitergibt. Der Beatmungskreislauf 50 ist über Anschlusselemente 56 und ein Schlauchsystem mit dem Patienten 30 verbunden. Das Schlauchsystem besteht aus einem Y-Stück 51 mit einem Absaugeanschluss 53, einem inspiratorischen Schlauchstück 57 und einem exspiratorischen Schlauchstück 55. Über die Absaugeleitung 59 ist die Messanordnung 70 mit dem Patienten 30 mittels des Y-Stücks 51 verbunden. Die Absaugeleitung 49 saugt nahe am Patienten 30 die Luft ab, und zwar vorzugsweise die ausgeatmete Luft, die dann in der Messanordnung 70 analysiert wird. Bei dieser Analyse wird u. a. der Kohlendioxidgehalt ermittelt und auf einer Anzeigeeinheit 73 zur Darstellung gebracht. In der Messanordnung 70 ist eine optische Messeinrichtung 75 vorhanden, die in Verbindung mit einer Betriebs- und Auswerteelektronik 71 ausgebildet ist, die Gaskonzentration an Kohlendioxid im gesammelten Messgas zu ermitteln. Im Beatmungsgerät 90 sind mehrere Elemente vorhanden, um eine Anästhesie und damit verbundene Beatmung des Patienten 30 vorzunehmen. Es ist eine Gasdosierung 97 vorgesehen, in die von außen über einen Zugang 11 Gase wie anschließend Sauerstoff und Lachgas und Narkosegase zugeführt werden können. Es ist ein Handbeatmungsbeutel 64 mit einer Zuleitung 62 vorgesehen. Der Handbeatmungsbeutel 64 ermöglicht dem Anästhesisten eine manuelle Beatmung und Anästhesie des Patienten 30. Im Gasweg an den Beatmungsantrieb 98 ist ein Narkosemittelverdunster 99 angeordnet, mit dessen Hilfe volatile Anästhetika wie beispielsweise Halothan in den inspiratorischen Luftstrom eindosiert und über das inspiratorische Schlauchstück 57 dem Patienten 30 zugeführt werden.

Es ist ein Modul 94 vorgesehen, das die Kohlendioxidentfernung mittels eines in dieser Figur 1 nicht näher gezeigten Kalkabsorbers vornimmt, das eine in dieser Figur 1 nicht näher gezeigte Narkosegasfortleitung enthält und einen Anschluss 61 zur Fortleitung des Narkosegases an die Umgebung bzw. an dazu in der Krankenhausinfrastruktur vorgesehene Gassammeleinrichtung vornimmt.

Weiterhin ist der Beatmungsantrieb 98 in dieser Ausführung als ein Radialverdichter mit der Funktionalität einer Druckquelle ausgebildet. Im Gasweg, anschließend an den Beatmungsantrieb 98, ist ein Narkosemittelverdunster 99 angeordnet, mit dessen Hilfe volatile Anästhetika wie beispielsweise Halothan in den inspiratorischen Luftstrom eindosiert und über das inspiratorische Schlauchstück 57 dem Patienten 30 zugeführt werden. An Sensorik und Aktuatorik sind ein inspiratorischer Durchflusssensor 93, ein exspiratorischer Durchflusssensor 95, ein exspiratorisches, gesteuertes Ventil 96 und ein exspiratorisch angeordneter Drucksensor 91 vorgesehen.

Weiterhin ist eine Betriebs- und Ansteuerungseinheit 900 vorgesehen, welche die Aktuatorik 96 und den Beatmungsantrieb 98 ansteuert und die Signale der Sensorik 91, 92, 93, 95 erfasst und weiter verarbeitet. Die zur Erfassung der Sensorik 91, 92, 93, 95 erforderlichen Datenverbindungen sind in dieser schematischen Darstellung nach Figur 1 nicht gezeigt.

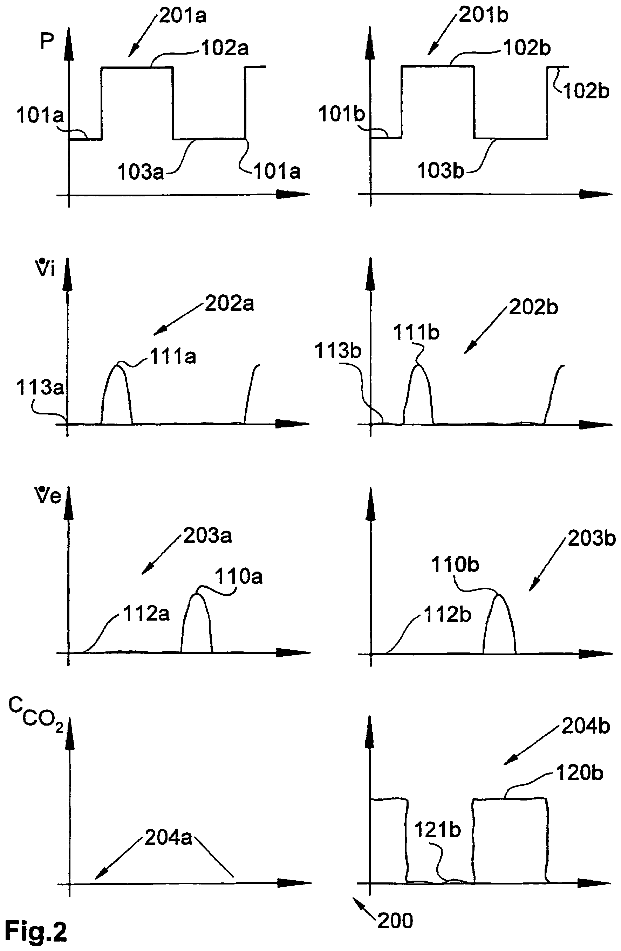

Die Figuren 2 bis 5 stellen einen zeitlichen Verlauf von Atemzyklen eines Patienten dar. Dargestellt werden in einer zeitlichen Synchronität Beatmungsdruck, Volumenstrom und die durch die Messanordnung absaugend gemessene Kohlendioxid-Konzentration (C_{CO₂}) über der Zeit.

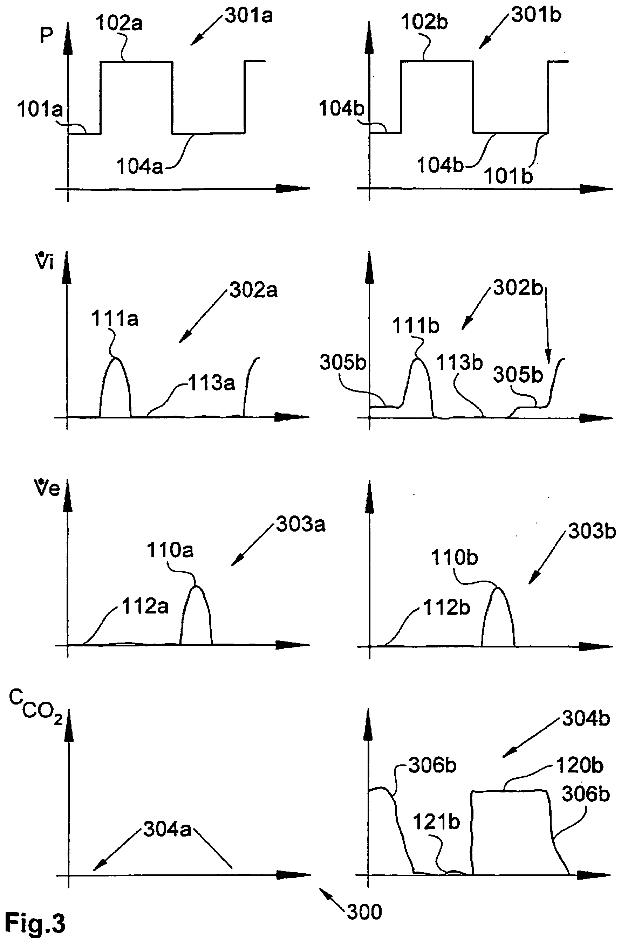

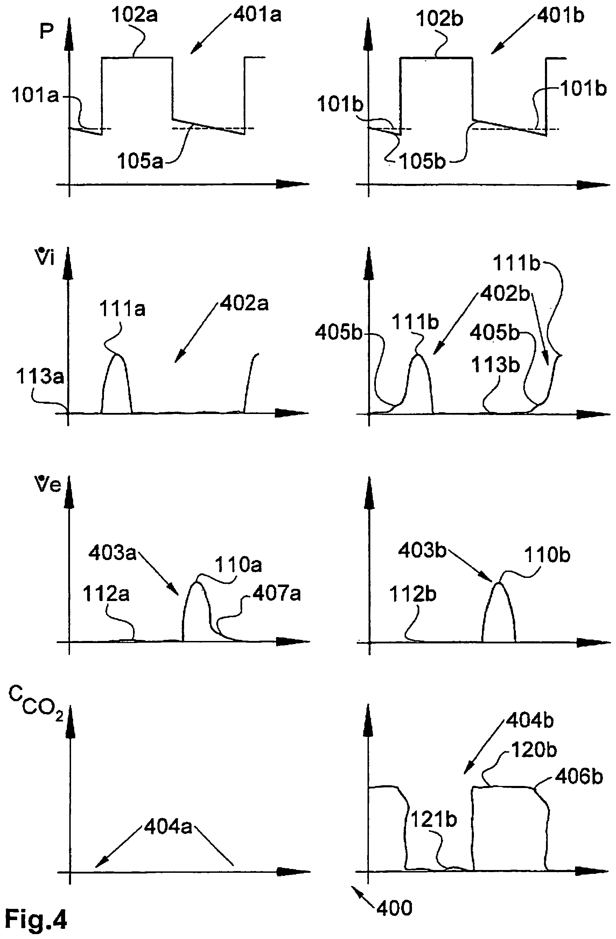

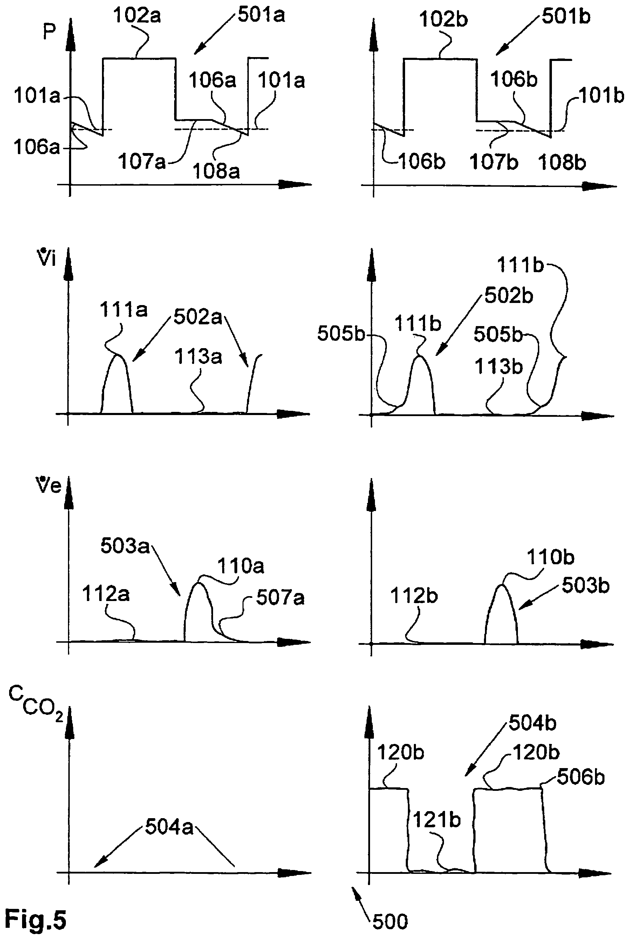

In der Fig. 2 wird eine Ausführung gezeigt, in der keine Ausregelung des PEEP-Drucks stattfindet. In der Fig. 3 wird eine Ausführung gezeigt, in der eine Ausführung des PEEP-Drucks stattfindet, wobei das PEEP-Niveau in der Exspirationsphase konstant gehalten wird. In der Fig. 4 wird eine Ausführung gezeigt, in der der PEEP-Druck ausgeregelt wird, wobei die Ausregelung nach einer fallenden Solldruck-Rampe angesteuert wird. In der Fig. 5 ist eine erste Variante nach Fig. 4 dargestellt, in der der PEEP-Druck ausgeregelt wird, wobei die Ausregelung in einem ersten Zeitabschnitt auf einem konstanten Niveau erfolgt, in einem zweiten Zeitabschnitt, anschließend an den ersten Zeitabschnitt, die Ausregelung nach einer fallenden Solldruck-Rampe erfolgt.

Die Figuren 2 bis 5 werden nun in einer gemeinsamen, einleitenden Figurenbeschreibung näher beschrieben. Weiterhin werden die Gemeinsamkeiten und Unterschiede in den Figuren 2, 3, 4, 5 ausgeführt. Es ist jeweils in einer Anordnung 200, 300, 400, 500 von sechs Diagrammen ein zeitlicher Verlauf des Beatmungsdrucks (P) des inspiratorischen Volumenstroms (V̇ₑ), des exspiratorischen Volumenstroms (V̇ₑ) und der Kohlendioxid-Konzentration (C_{CO₂}) gezeigt. Die drei linksseitig untereinander angeordneten Diagramme zeigen den schematischen Verlauf des Beatmungsdrucks (P), des inspiratorischen Volumenstroms (V̇ᵢ), des exspiratorischen Volumenstroms (V̇ₑ) und der Kohlendioxid-Konzentration (C_{CO₂}), gemessen durch die Messanordnung 70 (Fig. 1), bei nicht aktivierter Volumenstrom-Absaugung durch die Absaugeleitung 59 (Fig. 1) mittels der Pumpe 77 (Fig. 1) in die Messanordnung 70 (Fig. 1). Die drei rechtsseitig untereinander angeordneten Diagramme zeigen den schematischen Verlauf des Beatmungsdrucks (P), der Volumenströme (V̇ᵢ) und (V̇ₑ) sowie den Verlauf der Kohlendioxid-Konzentration (C_{CO2} ), gemessen in der Messanordnung 70 (Fig. 1) bei aktivierter Volumenstrom-Absaugung durch die Absaugeleitung 59 (Fig. 1) mittels der Pumpe 77 (Fig. 1) in die Messanordnung 70 (Fig. 1).

Die linksseitig angeordneten Diagramme (P, V̇ᵢ V̇ₑ , C_{CO₂}) sind durch die Verwendung des Index a zugehörigen Bezugsziffern kenntlich gemacht. Die rechtsseitig angeordneten Diagramme sind durch die Verwendung des Index b zugehörigen Bezugsziffern kenntlich gemacht. In dieser gemeinsamen Figurenbeschreibung sind die Bezugsziffern für die Diagramme derart gewählt, dass anhand der Bezugsziffern des Diagramms eine Zuordnung zu der zugehörigen Figur gegeben ist. In den Figuren 2 bis 5 identisch vorhandene Elemente sind mit den gleichen Bezugsziffern in allen Figuren 2, 3, 4, 5 versehen.

So beginnen die Bezugsziffern der Diagramme (P, V̇ᵢ, V̇ₑ, C_{CO₂}) für die Fig. 2 mit 200, für die Fig. 3 mit 300, für die Fig. 4 mit 400 und für die Fig. 5 mit 500. In den Figuren sind in den Diagramm-Zusammenschauen 200, 300, 400, 500 exspiratorische Druckverläufe (P) 201a ohne aktivierte Volumenstrom-Absaugung 59 (Fig. 1) und exspiratorische Druckverläufe 201 b mit aktivierter Volumenstrom-Absaugung 59 (Fig. 1) durch die Messanordnung 70 (Fig. 1). Weiterhin sind in den Diagramm-Zusammenschauen 200, 300, 400, 500 inspiratorische Volumenstromverläufe (V̇ᵢ) 202a, 302a, 402a, 502a und exspiratorische Volumenstromverläufe (V̇ₑ) 203a, 303a, 403a, 503a ohne aktivierte Volumenstrom-Absaugung 59 (Fig. 1) sowie inspiratorische Volumenstromverläufe (V̇ᵢ) 202b, 302b, 402b, 502b und exspiratorische Volumenstromverläufe (V̇ₑ) 203b, 303b, 403b, 503b mit aktivierter Volumenstrom-Absaugung 59 (Fig. 1) zeitlich korrespondierend zu den Druckverläufen (P) 201a, 201 b, 301 a, 301b, 401 a, 401 b, 501 a, 501 b gezeigt.

Zu den Druck- und Volumenstromverläufen korrespondierend, aber bedingt durch die Volumenstrom-Absaugung mit einem zeitlich Verzug, werden die Kohlendioxidkonzentrationen (C_{CO₂}) 204a, 304a, 404a, 504a ohne aktivierte Volumenstrom-Absaugung und die Kohlendioxidkonzentrationen (C_{CO₂}) 204b, 304b, 404b, 504b mit aktivierter Volumenstrom-Absaugung gezeigt.

Bei nicht aktivierter Volumenstrom-Absaugung oder ohne einen Anschluss der Messanordnung 70 (Fig. 1) am Beatmungskreislauf 50 (Fig. 1) am Patienten 30 (Fig. 1) sind keine Kohlendioxid-Messsignale vorhanden. Daher sind in den Diagrammen, welche linksseitig in den Figuren 2 bis 5; gekennzeichnet mit dem Index a, keine Verläufe der Kohlendioxidkonzentrationen (C_{CO₂}) 204a, 304a, 404a, 504a sichtbar. Dementsprechend sind die Diagramme 204a, 304a, 404a, 504a nur der Übersichtlichkeit und Vollständigkeit halber gezeigt.

In der Fig. 2 sind die Verläufe 201a, 201 b, 202a, 202b, 203a, 203b, 204a, 204b einer technischen Umsetzung eines Anästhesiegerätes 90 (Fig. 1) mit einer Messanordnung 70 (Fig. 1) in der Diagrammzusammenschau 200 gezeigt, bei welcher der Druck, insbesondere der verbleibende Druck in der Exspirationsphase nicht nachgeregelt wird. Es sind in den Verläufen des Drucks 201 a, 201 b exspiratorische Druckniveaus 101 a, 101 b als ungeregelter Verlauf einer 3a, einer 3b in Form eines Soll-Wertes oder eines Stell-Wertes dargestellt. Die in dieser Diagrammzusammenschau 200 dargestellten Verläufe von Kohlendioxidkonzentrationen (C_{CO₂}) 204 sowie die Verläufe der in- und exspiratorischen Volumenströme (V̇ᵢ, V̇ₑ) 202a, 202b, 203a, 203b sind auf Messungen basierende Ist-Werte. Die korrespondierenden Verläufe des Volumenströme (V̇ᵢ , V̇ₑ) 202a, 202b, 203a, 203b weisen in dieser Diagrammzusammenschau 200 keine Beeinflussung durch die Volumenstrom-Absaugung auf. Die Maximalniveaus 111a, 111b und die, Basisniveaus 113a, 113B des Inspirations-Volumenstroms (V̇ᵢ) 202a, 202b sowie auch die Maximalniveaus 110a, 110b und die Basisniveaus 112a, 112b des Exspirations-Volumenstroms (V̇ₑ) 203a, 203b entsprechen jeweils dem zugehörigen Verlauf des in- und exspiratorischen Druckniveaus 102a; 102b, 103a, 103b in den Druckverläufen 201 a, 202b.
Die Kohlendioxidkonzentration (C_{CO₂}) entspricht mit Basisniveau 121b und Maximalniveau 120b ebenfalls dem Druckverlauf 202b, ohne dass das Maximalniveau 120b im zeitlichen Verlauf der Exspiration nennenswerte Unstetigkeiten oder Signalverrundungen aufweist.

In der Fig. 3 sind die Verläufe 301a, 301b, 302a, 302b, 303a, 303b, 304a, 304b einer technischen Umsetzung eines Anästhesiegerätes 90 (Fig. 1) und einer Messanordnung 70 (Fig. 1) in der Diagrammzusammenschau 300 gezeigt, bei welcher der Druck während der Inspirationsdauer und während der Exspirationsdauer, insbesondere der positive endexspiratorische Druck (PEEP) in den Exspirationsphäsen nach einem konstanten Verlauf 104a, 104b ausgeregelt wird. Durch diese Regelung werden Leckagen, wie durch die aktivierte Volumenstrom-Absaugung 59 (Fig. 1) der Messanordnung 70 (Fig. 1), wie auch Leckagen im Beatmungskreislauf 50 (Fig. 1) und in der Gaszufuhr 51, 53, 54, 55, 56, 57 (Fig. 1) zum Patienten 30 (Fig. 1) kompensiert. Die Darstellung der Druckverläufe 301a, 301 b stellen in dieser Diagrammzusammenschau 300 ebenso wie die Verläufe der Volumenströme (V̇ᵢ, V̇ₑ) 302a, 302b, 303a, 303b und die Verläufe der Kohlendioxidkonzentration (C_{CO₂}) 304b mithilfe von Sensorik ermittelten Messwerten basierende zeitliche Abläufe dar. Der Verlauf des Exspirations- und Inspirations-Volumenstroms (V̇ᵢ , V̇ₑ) 303a, 302a mit Basisniveau 112a, 113a und Maximalniveau 111a, 110a ohne. Aktivierung der Volumenstrom-Absaugung zur Messanordnung 70 (Fig. 1) weisen keine wesentlichen Unterschiede zu den Verläufen 203 nach Fig. 2 auf. Der Exspirations-Volumenstromverlauf (V̇ₑ) 303b mit Maximalniveau 112b und Basisniveau 110b weist bei aktivierter Volumenstrom-Absaugung keine Unterschiede zum Exspirations-Volumenstromverlauf (V̇ₑ) 303a, 112a, 110a ohne aktivierte Volumenstrom-Absaugung auf. Der Inspirations-Volumenstrom (V̇ᵢ) zeigt neben dem Maximalniveau 111 b und dem Basisniveau 113b eine Abweichung 305b am Ende der Exspirationsphase auf. Diese Abweichung 305b ergibt sich durch die Volumenstrom-Absaugung selbst. Eine Menge an Gas wird aus dem Beatmungskreislauf 50 (Fig. 1) durch die Volumenstrom-Absaugung entfernt. Durch die Nachregelung des PEEP durch den Beatmungsantrieb 98 (Fig. 1), angesteuert durch die Betriebs- und Ansteuerungseinheit 900 (Fig. 1), wird inspiratorisches Gas wieder zugeführt, welches bei Durchströmung des inspiratorischen Durchflusssensors 91 (Fig. 1) erfasst wird und damit im Verlauf 302 als Abweichung 305b in Form einer zusätzlichen Durchflussmenge 305b am Ende der Exspiration sichtbar wird. Diese zusätzliche Durchflussmenge 305b bewirkt, dass am Y-Stück 51 (Fig. 1) die vom Patienten 30 (Fig. 1) in der Exspiration ausgeatmete Gasmenge mit frischem Inspirationsgas vermischt wird. Diese Vermischung bedingt eine Verringerung der Kohlendioxidkonzentration ( C_{CO₂}) am Y-Stück 51 (Fig. 1), da durch die Kohlendioxidentfernung im Modul 94 (Fig. 1) des Anästhesiegerätes 90 (Fig. 1) der Kohlendioxid aus der Ausatemluft des Patienten 30 (Fig. 1) aus dem Gas entfernt wird und somit kohlendioxidfreies Gas inspiratorisch zum Patienten 30 (Fig. 1) geliefert wird. Diese Verringerung der Kohlendioxidkonzentration (C_{CO₂}) wird im Verlauf der Kohlendioxidkonzentration ( C_{CO₂}) 304b als ein Abfall des Konzentrationsverlaufs 306b am Ende der Exspiration vom Maximalniveau 120b der exspiratorischen Kohlendioxidkonzentration (C_{CO₂}) sichtbar.

In der Fig. 4 und in der Fig. 5 sind die Verläufe 401 a, 401 b, 402a, 402b, 403a, 403b, 404a, 404b, 501 a, 501 b, 502a, 502b, 503a, 503b, 504a, 504b einer technischen Umsetzung eines Anästhesiegerätes 90 (Fig. 1) und einer Messanordnung 70 (Fig. 1) in den Diagrammzusammenschauen 400, 500 gezeigt, bei welchen der positiv-endexspiratorische Druck (PEEP) im Unterschied zur technischen Umsetzung nach der Fig. 3 nicht konstant geregelt wird, sondern in einer Art, dass der geregelte Druckwert am Beginn der Exspiration größer ausgeregelt wird, als der geregelte Druckwert am Ende der Exspiration. In den technischen Ausführungsformen nach den Fig. 4 und 5 wird der Unterschied im Druckniveau zwischen dem Beginn und dem Ende der Exspirationsphase dadurch erreicht, dass der PEEP-Druck während der Exspirationsphase im Verlauf vermindert wird. Diese Verminderung des PEEP über die Dauer der Exspirationsphase kann, wie in Fig. 4 in der Diagrammzusammenschau 400 ersichtlich ist, gleich zu Beginn gemäß einer fallenden Rampe 105a, 105b erfolgen. Die Verminderung kann aber auch nach einem Verlauf 106a, 106b gemäß der Fig. 5 und der Diagrammzusammenschau 500 mit einem konstanten Anteil 107a, 107b zu Beginn der Exspirationsphase und einem während der Dauer der Exspirationsphase einsetzenden, abfallenden Anteil 108a, 108b umgesetzt werden.

Der Verlauf der Druckverläufe 105a, 106a, 105b, 106b in der Exspirationsphase nach den Figuren 4 und 5 wird in der Ausgestaltung der Niveaus am Beginn sowie am Ende der Exspirationsphase und auch in der Ausgestaltung des abfallenden und auch des konstanten Anteils des Verlaufs auf Basis des exspiratorischen Druckniveaus 101 a, 101 b ermittelt. Das in den Figuren 4 und 5 als gestrichelt eingezeichnete exspiratorische Druckniveau 101 a, 101 b entspricht im Mittel den Verläufen 105a, 105b, 106a, 106b, so dass sich in der Druckbilanz des Drucks (PEEP) am Patienten je Exspirationsphase kein Unterschied für den Patienten 30 (Fig. 1) gegenüber einer konstanten PEEP-Ausregelung 104a, 104b nach der Fig. 3 ergibt. Dadurch, dass das Druckniveau während der Exspiration reduziert wird, ist es dem Patienten 30 (Fig. 1) ermöglicht, gegen Ende der Exspiration bis nahe zu dem Beginn der nächsten Inspiration weiterhin auszuatmen, da das Druckniveau in der Lunge 26 (Fig. 1) des Patienten 30 (Fig. 1) ebenfalls gemäß dem Verlauf der fallenden Rampe 105a, 105b, 106a, 106b, 108a, 108b abgesenkt wird. Diese zusätzliche und auch länger andauernde Ausatmung gelangt zum Y-Stück 51 (Fig. 1) und über die Absaugeleitung 59 (Fig. 1) zur Messanordnung 70 (Fig. 1). Damit wird dort eine Vermischung von ausgeatmetem Gas mit aufgrund der Volumenstrom-Absaugung nachgeregeltem, frischem Inspirationsgas am Y-Stück 51 (Fig. 1) vermieden, so dass bei aktivierter Volumenstrom-Absaugung im Gegensatz zu einer konstanten Ausregelung 104a, 104b des PEEP gemäß der Fig. 3 die Verringerung der Kohlendioxidkonzentration (C_{CO₂}) im Könzentrationsverlauf 404b, 504b weniger signifikant als ein Abfall 406b oder nur noch unwesentlich als ein Abfall 506b erkennbar ist. Damit ergibt sich gleichsam eine gleichbleibende Anzeigesituation hinsichtlich der angezeigten Kohlendioxidkonzentration (C_{CO₂}) über die gesamte Dauer der Exspiration hinweg für den Anwender.

In der Fig. 5 ergibt sich im Verlauf 106a, 106b durch den zu Beginn vorhandenen konstanten Anteil 107a, 107b des Druckverlaufs ein steilerer Gradient des fallenden Anteils 108a, 108b des Druckverlaufs gegenüber dem Verlauf 105a, 105b in Fig. 4. Dieser steilere Gradient 108a, 108b bewirkt auch am Ende der Exspirationsphase noch eine Ausatmung des Patienten, so dass die Verminderung am Y-Stück 51 (Fig. 1) mit nachgeregeltem frischem Inspirationsgas noch weniger stattfinden kann. Dies ist aus den Unterschieden zwischen den abfallenden Verläufen 406b, 506b zwischen den technischen Umsetzungen nach. Fig. 4 und Fig. 5 ersichtlich. In den rechtsseitigen Diagrammen in den Figuren 4 und 5 der Volumenströme (V̇ᵢ, V̇ₑ) 402b, 403b, 502b, 503b sind gegenüber der technischen Umsetzung mit ungeregeltem, PEEP nach Fig. 2 nur geringe Unterschiede zu den Verläufen 202b, 203b ersichtlich. Dies ergibt sich daraus, dass die Nachregelung des PEEP genau wie in der Fig. 3 am Ende der Exspiration einen inspiratorischen Volumenstrom (V̇ᵢ) verursacht, welcher sich in Form einer Abweichung oder eines Vorlaufs 405b, 505b als eine zusätzliche Durchflussmenge neben den Maximalniveaus 111 b und den Basisniveaus 113b des Inspirations-Volumenstroms darstellt. Bei nicht aktivierter Volumenstrom-Absaugung bewirkt der abfallende Verlauf 401 a, 501 b in den linksseitigen Diagrammen in den Figuren 4 und 5, dass die vom Patienten 30 (Fig. 1) am Ende der Exspiration noch ausgeatmete Gasmenge nicht am Y-Stück 51 (Fig. 1) der Anordnung 70 (Fig. 1) abgesaugt wird, sondern über das exspiratorische Schlauchstück 55 (Fig. 1) in das Anästhesiegerät 90 (Fig. 1) gelangt und dort vom exspiratorischen Durchflusssensor 95 (Fig. 1) erfasst wird. Dies ist in den Diagrammzusammenschauen 400, 500 zu den Figuren 4 und 5 linksseitig als ein Nachlauf des Exspirations-Volumenstroms 407a, 507a im Verlauf der exspiratorischen Durchflussmenge 402a, 502a ersichtlich.

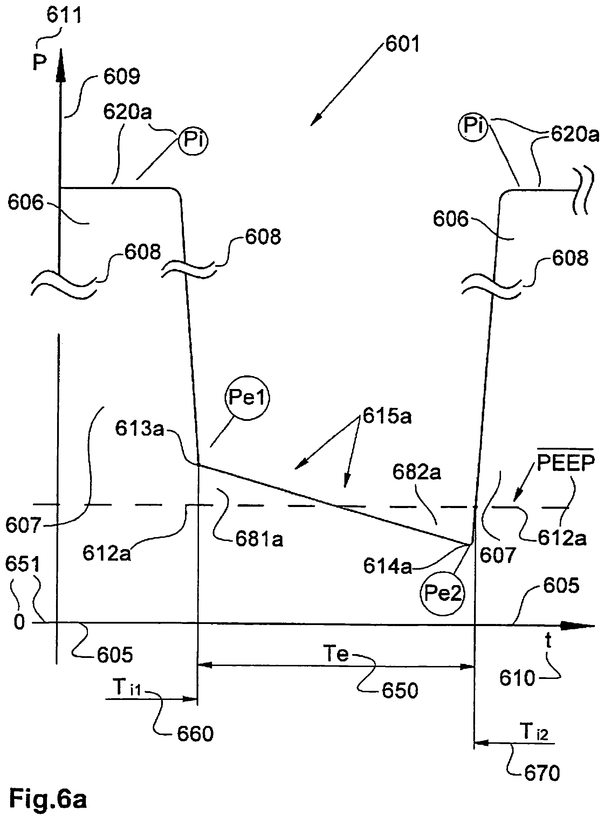

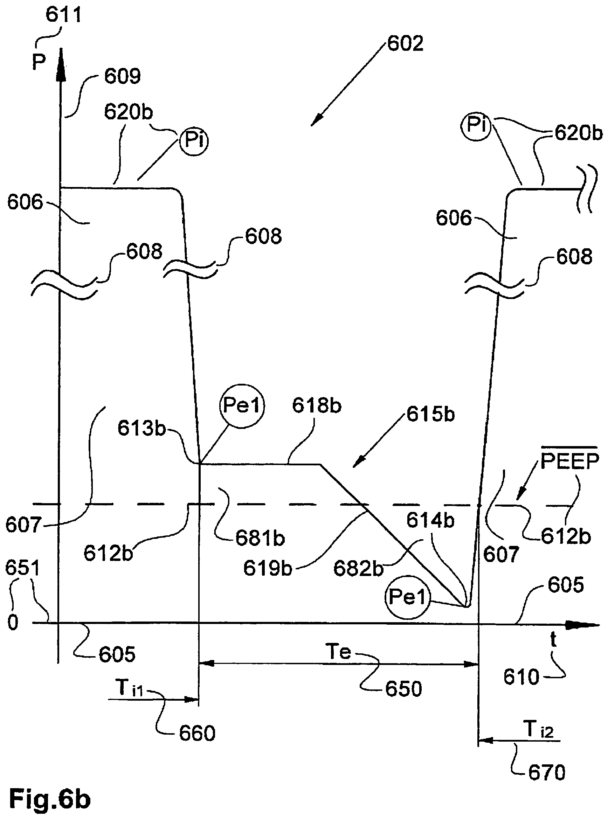

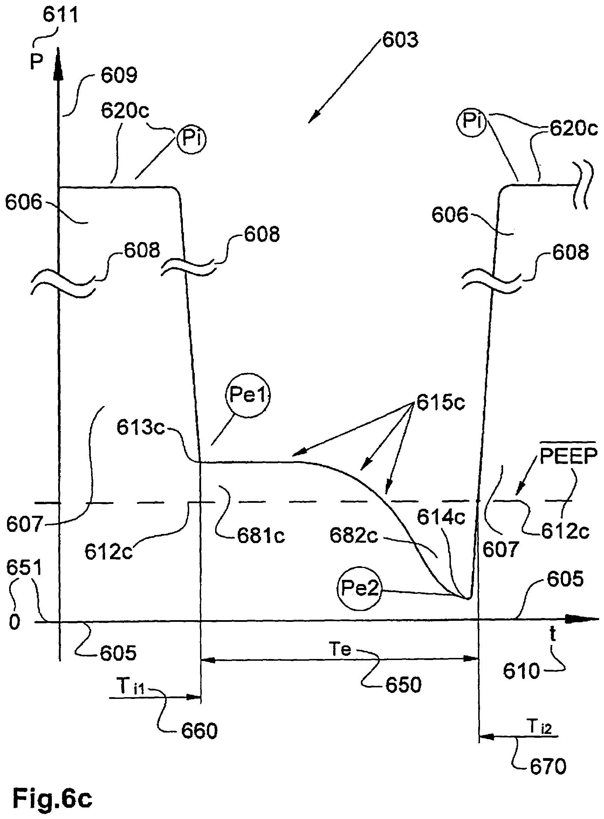

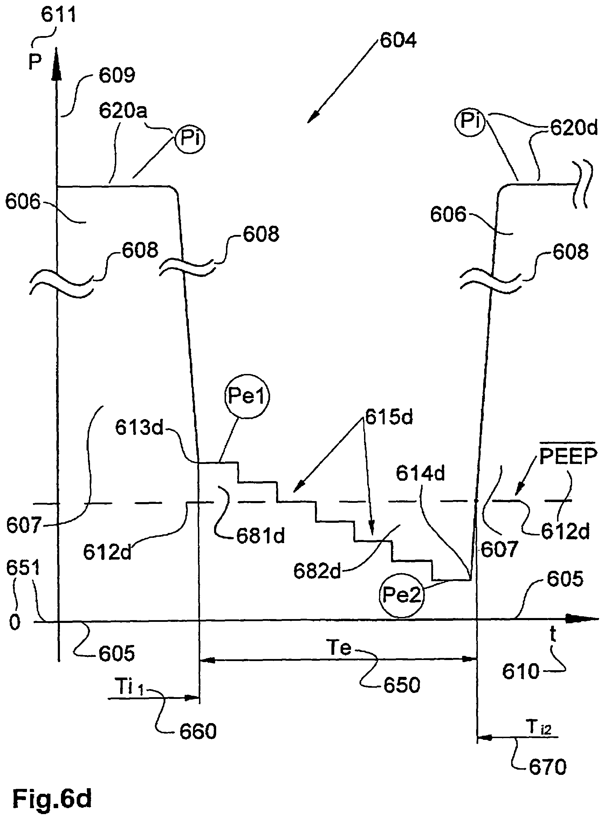

In den Figuren 6a, 6b, 6c und 6d sind technische Umsetzungsvarianten nach den Figuren 4 und 5, sowie weitere technische Umsetzungsvarianten gezeigt, in denen der geregelte positiv-endexspiratorische Druck (PEEP) am Ende der Exspirationsphase gegenüber dem Beginn vermindert ist.

Die Figuren 6a, 6b, 6c, 6d zeigen Varianten der Regelung des positiv-endexspiratorischen Drucks (PEEP) gemäß den Druckverläufen in den Figuren 4 und 5. Gleiche Elemente in den Figuren 6a, 6b, 6c, 6d sind mit den gleichen Bezugsziffern für die gleichen Elemente in den Figuren 2, 3, 4, 5 versehen. Die Fig. 6a zeigt einen schematischen Druckverlauf 601 am Patienten gemäß dem Druckverlauf 401 b in der Fig. 4. Die Fig. 6b zeigt einen schematischen Druckverlauf 602 am Patienten gemäß dem Druckverlauf 501 b in der Fig. 5. Die Fig. 6c zeigt einen Druckverlauf 603 am Patienten in einer abgewandelten Form gemäß dem Druckverlauf 501 b in der Fig. 5. Die Fig. 6d zeigt einen Druckverlauf 604 am Patienten in einer abgewandelten Form gemäß dem Druckverlauf 401 b in der Fig. 4.

Die Darstellungen der Druckverläufe 601, 602, 603, 604 in den Figuren 6a, 6b, 6c, 6d sind in keiner Weise abschließend. Insbesondere Kombinationen der abgewandelten Formen 603, 604 miteinander und/oder mit den Druckverläufen 601, 602 sind im Sinne der vorliegenden Erfindung mit umfasst.

Die Figuren 6a, 6b, 6c, 6d werden nun in einer gemeinsamen Figurenbeschreibung näher in den Gemeinsamkeiten und mit Verdeutlichung der Unterschiede zueinander in den technischen Ausgestaltungen der Regelung des positiv-endexspiratorischen Drucks (PEEP) näher erläutert.

In den Figuren 6a, 6b, 6c, 6d werden für identische Elemente jeweils identische Bezugsziffern verwendet. Die an/in den Bezugsziffern zusätzlich verwendeten Indizes a, b, c, d dienen der Unterscheidbarkeit grundsätzlich identischer Bezugsziffern und Merkmale in den Figuren 6a, 6b, 6c, 6d. Die Verwendung des Index a bezieht sich auf Elemente der Fig. 6a. Die Verwendung des Index b bezieht sich auf Elemente der Fig. 6b. Die Verwendung des Index c bezieht sich auf Elemente der Fig. 6c. Die Verwendung des Index d bezieht sich auf Elemente der Fig. 6d. In den gezeigten Druckverläufen 601, 602, 603, 604 der Figuren 6a, 6b, 6c, 6d sind Druckverläufe beginnend am Ende einer Inspiration mit einem ersten Anteil 1 Tᵢ₁ 660 mit einem inspiratorischen Druckniveau Pᵢ 620, einer daran anschließenden Exspirationszeitdauer Tₑ 650 und einem daran folgenden Anteil 2 Tᵢ₂ 670 der nächstfolgenden Inspiration mit dem inspiratorischen Druckniveau Pᵢ 620 entlang der Abszisse (x) 605 über der Zeit 610 aufgetragen.

In der gezeigten Exspirationszeitdauer Tₑ 650 sind exspiratorische, abfallende Druckverläufe 615a (Fig. 6a), 615b (Fig. 6b), 615c (Fig. 6c) und 615d (Fig. 6d) dargestellt. Die schematischen Druckverläufe 601, 602, 603, 604 sind als Druck 611 auf einer Ordinate (y) 609 skaliert. Die Ordinate 609 ist durch ein Trennungszeichen 608 in zwei Abschnitte geteilt. Der erste Abschnitt der Ordinate 606 skaliert die inspiratorischen Druckniveaus 620 in den Exspirationszeitdauern Tᵢ₁ 660 und Tᵢ₂ 670. Der zweite Abschnitt der Ordinate 607 ist in einer abweichenden Skalierung des positiv-endexspiratorischen Drucks (PEEP) in der Exspirationszeitdauer Tₑ 650 angepasst, um die exspiratorischen, abfallenden Druckverläufe in grafischer Form in geeigneter Weise darstellbar zu machen. Die inspiratorischen Druckniveaus 620 sind in den Figuren 6a, 6b, 6c, 6d im Betrag identisch gewählt. Die Skalierung der Ordinate 609 und der Abschnitte der Ordinate 606, 607 sind in den Figuren 6a, 6b, 6c, 6d identisch gewählt und dargestellt. Als Bezug zur Skalierung der Ordinate 609 ist ein Null-Niveau 651 in den Figuren 6a, 6b, 6c, 6d eingezeichnet. Weiterhin ist in Form einer gestrichelten Linie ein mittlerer Wert des positiv-endexspiratorischen Drucks P̅E̅E̅P̅ 612a, 612b, 612c, 612d mit Bezug zum Null-Niveau 651 eingezeichnet. Von diesem Mittelwert (P̅E̅E̅P̅) 612 des positiv-endexspiratorischen Drucks werden die Startwerte Pₑ₁ 613a, 613b, 613c, 613d und die Endwerte Pₑ₂ 614a, 614b, 614c, 614d des exspiratorischen Drucks am Beginn und Ende der Exspirationszeitdauer Tₑ 650 abgeleitet und bestimmt. Diese Bestimmung von Pₑ₁ 613a, 613b, 613c, 613d und Pₑ₂ 614a, 614b, 614c, 614d erfolgt in den technischen Ausführungsformen der Figuren 6a, 6b, 6c, 6d auf Basis des vorbestimmten Mittelwerts P̅E̅E̅P̅ 612, der Exspirationszeitdauer Tₑ 650 sowie der jeweiligen Verlaufsform der Druckausregelung in der jeweiligen Fig. 6a, 6b, 6c, 6d. Der mittlere Wert P̅E̅E̅P̅ 612 des endexspiratorischen Drucks wie auch die Exspirationszeitdauer Tₑ 650, das inspiratorische Druckniveau Pᵢ 620 ergeben sich unterschiedlich für verschiedene Typen von Patienten, wie sie in der Beschreibung in Tabelle 1 und in Tabelle 2 dargelegt sind.

In der Fig. 6a ist der Druckverlauf des PEEP über die Exspirationszeitdauer Tₑ 650 als eine lineare abfallende Rampe 615a gezeigt, die über die gesamte Exspirationsdauer Tₑ 650 abfallend ausgeführt ist.

In der Fig. 6b ist der Druckverlauf des PEEP über die Exspirationszeitdauer Tₑ 650 als ein zweiteiliger Funktionsverlauf 615b beginnend mit einem Zeitabschnitt des Verlaufs mit konstantem Druckniveau 618b und einem daran anschließenden Zeitabschnitt des Verlaufs mit abfallendem Druck 619b ausgeführt.

In der Fig. 6c ist der Druckverlauf des PEEP über die Exspirationszeitdauer Tₑ 650 als eine abgewandelte Form des zweiteiligen Funktionsverlaufs 615b nach der Fig. 6b ausgeführt, wobei konstanter und abfallender Zeitabschnitt nach einem progressiv abfallenden Funktionsverlauf 615c stufenlos ineinander übergehen. Ein solcher progressiv abfallender Funktionsverlauf 615c lässt sich vorzugsweise mittels Potenzfunktionen, exponentiellen oder logarithmischen Funktionen, sowie gebrochen-rationalen Funktionen gestalten oder in besonderer Weise mittels Kombinationen aus Potenzfunktionen, exponentiellen, logarithmischen oder gebrochenen rationalen Funktionen geeignet realisieren. Ein progressiver Abfall des PEEP gegen Ende der Exspirationsphase bewirkt, dass der Patient 30 (Fig. 1) über die gesamte Exspiration ausgeatmete, kohlendioxidhaltige Luft zum Y-Stück 53 (Fig. 1) strömen lässt. Damit ergibt sich am Y-Stück 53 (Fig. 1) keine Vermischung mit frischem Inspirationsgas, so dass es zu keinem Abfall der Kohlendioxidkonzentration (C_{CO₂}) wie bei einer Ausregelung auf einem gleichbleibend konstantem PEEP, wie in der Fig. 3 dargestellt, kommt. Es ergibt sich durch den progressiven Abfall 615c vielmehr der Effekt, dass sich kein wesentlicher Abfall der Kohlendioxidkonzentration (C_{CO₂}) am Ende der Exspiration ergibt, der vergleichbar ist mit dem Abfall, wie es in der Fig. 5 im Verlauf der exspirätorischen CO₂-Konzentration 504b (Fig. 5) dargestellt ist.

In der Fig. 6d ist ein Druckverlauf nach Fig. 6a in einer abgewandelten Form dargestellt. Der abfallende Verlauf 615d des exspiratorischen Drucks während der Exspirationszeitdauer Tₑ 650 ist als ein unstetiger Verlauf ausgebildet. Das bedeutet, dass der Abfall des PEEP während der Exspiration nicht als ein stetiger Funktionsverlauf, sondern als ein unstetiger, in Stufen abfallender Verlauf 615d durch die Druckregelung in der Betriebs- und Auswerteeinheit 900 (Fig. 1) realisiert ist. Eine solche gestufte oder auch schrittweise Absenkung des PEEP ergibt sich beispielsweise durch eine Digitalisierung und/oder Quantisierung in digitalen und/oder binären Recheneinheiten (Mikrokontroller, Prozessor, digitale Signalprozessoren) auf Basis der in diesen Systemen eingesetzten Bit-Auflösungen.

In den Figuren 6a, 6b, 6c, 6d sind die mittleren PEEP-Werte (P̅E̅E̅P̅) 612a, 612b, 612c, 612d als ein erster Vorgabewert, sowie die Dauer der Exspirationsphase Tₑ 650 als zweiter Vorgabewert gewählt worden. In Verbindung mit der jeweils gewählten Form des Verlaufs des Druckabfalls 615a, 615b, 615c, 615d werden der Startwert (Pₑ₂) des exspiratorischen Drucks 613a, 613b, 613c, 613d und der Endwert des exspiratorischen Drucks (Pₑ₂) 614a, 614b, 614c, 614d festgelegt. Diese Festlegung erfolgt in den Figuren 6a, 6b, 6c, 6d dergestalt, dass die Startwerte (Pₑ₁) 613a, 613b, 613c, 613d und die Endwerte (Pₑ₂) 614a, 614b, 614c, 614d in Verbindung mit dem Verlauf 615a, 615b, 615c, 615d so gewählt werden, dass eine erste Fläche 681 a, 681 b, 681 c, 681 d, aufgespannt zwischen dem mittleren PEEP (P̅E̅E̅P̅) 612a, 612b, 612c, 612d und dem Verlauf 615a, 615b, 615c, 615d oberhalb des mittleren PEEP (P̅E̅E̅P̅) 612a, 612b, 612c, 612d und eine zweite Fläche 682a, 682b, 682c, 682d, aufgespannt zwischen dem mittleren PEEP (P̅E̅E̅P̅) 612a, 612b, 612c, 612d und dem Verlauf 615a, 615b, 615c, 615d unterhalb des mittleren PEEP (P̅E̅E̅P̅) 612a, 612b, 612c, 612d im Flächeninhalt übereinstimmen. Durch diese Festlegung ergibt sich, unter Realisierung einer fallenden Druckrampe nach den Figuren 6a, 6b, 6c, 6d, ein für den Patienten 30 (Fig. 1) positiv-endexspiratorischer Druck (PEEP), der im Mittel dem gleichen positiv-endexspiratorischen Druck (PEEP) wie bei einer konstanten Ausregelung des PEEP über die Exspirationsphase, wie in Fig. 2 dargestellt, entspricht, mit dem Vorteil, dass die gemessene Kohlendioxidkonzentration (C_{CO₂}) zum Ende der Exspirationsphase nicht oder nicht wesentlich abfällt.

### Bezugszeichenliste

- 10: Anordnung
- 11: Zugang
- 30: Patient
- 50: Beatmungskreislauf
- 51: Y-Stück
- 53: Absauganschluss
- 54: Schlauchsystem
- 55: exspiratorisches Schlauchstück
- 56: Anschlusselemente
- 57: inspiratorisches Schlauchstück
- 59: Absaugeleitung
- 61: Anschluss
- 62: Zuleitung
- 64: Handbeatmungsbeutel
- 70: Messanordnung
- 71: Auswerte- und Betriebseinheit
- 73: Anzeigeelement
- 75: Messeinheit
- 77: Pumpe
- 79: Ausgang
- 80: Umgebung
- 90: Anästhesiegerät / Beatmungsgerät
- 91: exspiratorischer Drucksensor
- 92: inspiratorischer Drucksensor
- 93: inspiratorischer Durchflusssensor
- 94: Modul
- 95: exspiratorischer Durchflusssensor
- 96: Exspirationsventil
- 97: Gasdosierung
- 98: Beatmungsantrieb
- 99: Narkosemittelverdunster
- 900: Betriebs- und Ansteuerungseinheit
- 101a, b: exspiratorisches Druckniveau
- 102a, b: inspiratorisches Druckniveau
- 103a, b: ungeregelter Verlauf des exspiratorischen Druckniveaus (Sollwert)
- 104a, b: konstant geregelter Verlauf des exspiratorischen Druckniveaus (Istwert)
- 105a, b: nach einem ersten abfallenden Verlauf geregeltes exspiratorisches Druckniveau (Istwert)
- 106a, b: nach einem zweiten abfallenden Verlauf geregeltes exspiratorisches Druckniveau (Istwert)
- 107a, b: konstanter Anteil des Druckverlaufs
- 108a, b: fallender Anteil des Druckverlaufs
- 110a, b: Maximalwert des Exspirations-Volumenstroms
- 111a, b: Maximalniveau des Inspirations-Volumenstroms
- 112a, b: Basisniveau des Exspirations-Volumenstroms
- 113a, b: Basisniveau des Inspirations-Volumenstroms
- 120b: Maximalniveau der exspiratorischen Kohlendioxid-Konzentration (C_{CO₂})
- 121b: Basisniveau der exspiratorischen Kohlendioxid-Konzentration (C_{CO₂})
- 200: erste Diagrammzusammenschau
- 201a, b: erster Verlauf des Drucks am Patienten
- 202a, b: erster Verlauf des Inspirations-Volumenstroms
- 203a, b: erster Verlauf des Exspirations-Volumenstroms
- 204a, b: erster Verlauf der exspiratorischen Kohlendioxid-Konzentration (C_{CO₂})
- 300: zweite Diagrammzusammenschau
- 301a, b: zweiter Verlauf des Drucks am Patienten
- 302a, b: zweiter Verlauf des Inspirations-Volumenstroms
- 303a, b: zweiter Verlauf des Exspirations-Volumenstroms
- 304a, b: zweiter Verlauf der exspiratorischen Kohlendioxid-Konzentration (C_{CO₂})
- 305b: erste Abweichung des Inspirations-Volumenstroms am Ende der Exspiration, zusätzliche Durchflussmenge
- 306b: Abfall der Kohlendioxid-Konzentration (C_{CO₂}) am Ende der Exspiration
- 400: dritte Diagrammzusammenschau
- 401a, b: dritter Verlauf des Drucks am Patienten
- 402a, b: dritter Verlauf des Inspirations-Volumenstroms
- 403a, b: dritter Verlauf des Exspirations-Volumenstroms
- 404b: dritter Verlauf der exspiratorischen Kohlendioxid-Konzentration (C_{CO₂})
- 405b: Abweichung des Inspirations-Volumenstroms bei Ende der Exspiration, zusätzliche Durchflussmenge
- 406b: Abfall der Kohlendioxid-Konzentration (C_{CO₂}) am Ende der Exspiration
- 407a: Nachlauf des Exspirations-Volumenstroms
- 500: vierte Diagrammzusammenschau
- 501a, b: vierter Verlauf des Drucks am Patienten
- 502a, b: vierter Verlauf des Inspirations-Volumenstroms
- 503a, b: vierter Verlauf des Exspirations-Volumenstroms
- 504a, b: vierter Verlauf der exspiratorischen Kohlendioxid-Konzentration (C_{CO₂})
- 505b: Abweichung des Inspirations-Volumenstroms Am Ende der Exspiration, zusätzliche Durchflussmenge
- 506b: Abfall des Kohlendioxid-Konzentrationsverlaufs (C_{CO₂}) am Ende der Exspiration
- 507a: Nachlauf des Exspirations-Volumenstroms
- 601: schematischer Druckverlauf am Patienten
- 602: schematischer Druckverlauf am Patienten
- 603: schematischer Druckverlauf am Patienten
- 604: schematischer Druckverlauf am Patienten
- 605: Abszisse (x) (Zeitachse)
- 606: erster Abschnitt der Ordinate (y) (Druck)
- 607: zweiter Abschnitt der Ordinate (y) (Druck)
- 608: Trennungszeichen
- 609: Ordinate (y) (Druck)
- 610: Zeit
- 611: Druck
- 612a, b, c, d: P̅E̅E̅P̅, mittlerer Wert des positiv endexspiratorischen Drucks
- 613a, b, c, d: Startwert (Pₑ₁) des exspiratorischen Drucks
- 614a, b, c, d: Endwert (Pₑ₂) des exspiratorischen Drucks
- 615a, b, c, d: abfallender Verlauf des exspiratorischen Drucks während der Exspirationszeitdauer Tₑ
- 618b: Zeitabschnitt des exspiratorischen Druckverlaufs mit konstantem Druck
- 619b: Zeitabschnitt des exspiratorischen Druckverlaufs mit abfallendem Druck
- 620a, b, c, d: inspiratorisches Druckniveau Pᵢ
- 650: Tₑ, Exspirationszeitdauer
- 651: Null-Niveau des Druckverlaufs am Patienten
- 660: Tᵢ₁, Inspirationszeitdauer, Anteil 1
- 670: Tᵢ₂, Inspirationszeitdauer, Anteil 2
- 681a, b, c, d: erste Fläche des exspiratorischen Druckverlaufs während der Exspirationszeitdauer Tₑ
- 682a, b, c, d: zweite Fläche des exspiratorischen Druckverlaufs während der Exspirationszeitdauer Tₑ

## Patentansprüche

1. Anästhesie- oder Beatmungsgerät mit Steuerung eines endexspiratorischen Druckes am Ende einer Exspirationsphase (650) eines Patienten, wobei das Anästhesie- oder Beatmungsgerät einen Beatmungskreislauf (50) aufweist, in dem ein exspiratorischer Drucksensor (91), ein inspiratorischer Drucksensor (91), ein inspiratorischer Durchflusssensor (93), ein exspiratorischer Durchflusssensor (95), ein steuerbares exspiratorisches Ventil (96) und ein Beatmungsantrieb (98) angeordnet sind, und einer Betriebs- und Ansteuerungseinheit (900), die mit den vorhergenannten Komponenten in dem Beatmungskreislauf verbunden ist, **dadurch gekennzeichnet, dass** die Betriebs- und Ansteuerungseinheit (900) dazu eingerichtet ist, das steuerbare, exspiratorische Ventil (96) und den Beatmungsantrieb (98) in ihrem Betrieb so zu regeln, dass der Druckverlauf (401 a, 401 b, 501 a, 501 b) während der Exspirationsphase (650) vom Ende der Inspirationsphase (660) bis zum Beginn der nächsten, folgenden Inspirationsphase (670) von einem ersten oberen Druckwert (613a, 613b, 613c, 613d) zu einem ersten unteren Druckwert (614a, 614b, 614c, 614d) einen fallenden Verlauf (105a, 105b, 106a, 106b, 615a, 615b, 615c, 615d) mit höchstens stückweise konstanten Teilintervallen aufweist.

2. Anästhesie- oder Beatmungsgerät nach Anspruch 1, wobei die Betriebsund Ansteuerungseinheit (900) weiter dazu eingerichtet ist, den Betrieb des steuerbaren exspiratorischen Ventils (96) und des Beatmungsantriebs (98) so zu regeln, dass der fallende Verlauf vom ersten oberen Druckwert (613a, 613b, 613c, 613d) zum ersten unteren Druckwert (614a, 614b, 614c, 614d) einen linear fallenden Verlauf (601, 602, 604) und/oder einen nicht-linear fallenden Verlauf (603) aufweist.

3. Anästhesie- oder Beatmungsgerät nach Anspruch 1, wobei die Betriebsund Ansteuerungseinheit (900) weiter dazu eingerichtet ist, den Betrieb des steuerbaren exspiratorischen Ventils (96) und des Beatmungsantriebs (98) so zu regeln, dass der fallende Verlauf von einem ersten oberen Druckwert (613a, 613b, 613c, 613d) zu einem ersten unteren Druckwert (614a, 614b, 614c, 614dz) einen stetig fallenden Verlauf (601, 602, 603) und/oder einen Verlauf (604), der fallende und konstante Teilintervalle umfasst, aufweist.

4. Anästhesie- oder Beatmungsgerät nach Anspruch 1, wobei die Betriebsund Ansteuerungseinheit (900) weiter dazu eingerichtet ist, den Betrieb des steuerbaren exspiratorischen Ventils (96) und des Beatmungsantriebs (98) so zu regeln, dass der zumindest teilweise fallende Verlauf (108a, 108b) von einem ersten oberen Druckwert (613a, 613b, 613c, 613d) zu einem ersten unteren Druckwert (614a, 614b, 614c, 614d) nach einer Verzugszeit mit einem konstanten Druckniveau (107a, 107b) startet.

5. Anästhesie- oder Beatmungsgerät nach Anspruch 1, wobei die Betriebsund Ansteuerungseinheit (900) dazu eingerichtet ist, den ersten oberen Druckwert (613a, 613b, 613c, 613d) und den ersten unteren Druckwert (614a, 614b, 614c, 614d) in Abhängigkeit von Einstellungen des Beatmungs- oder Anästhesiegerätes zu bestimmen.

6. Anästhesie- oder Beatmungsgerät nach Anspruch 5, wobei die Betriebsund Ansteuerungseinheit (900) dazu eingerichtet ist, den ersten oberen Druckwert und den ersten unteren Druckwert festzulegen und dabei Einstellungen zu PEEP oder der Atemfrequenz und/oder des I/E-Verhältnisses heranzuziehen.

## Claims

1. An anaesthesia- or ventilator apparatus with control of an end-expiratory pressure at the end of an expiration phase (650) of a patient, wherein the anaesthesia- or ventilator apparatus has a ventilator circuit (50), in which an expiratory pressure sensor (91), an inspiratory pressure sensor (91), an inspiratory flow sensor (93), an expiratory flow sensor (95), a controllable expiratory valve (96) and a ventilator drive (98) are arranged, and an operating- and actuating unit (900), which is connected to the previously mentioned components in the ventilator circuit, **characterized in that** the operating- and actuating unit (900) is arranged to regulate the controllable, expiratory valve (96) and the ventilator drive (98) in their operation so that the pressure profile (401 a, 401 b, 501 a, 501 b) during the expiration phase (650) from the end of the inspiration phase (660) to the start of the next, following inspiration phase (670) has a decreasing profile (105a, 105b, 106a, 106b, 615a, 615b, 615c, 615d) from a first upper pressure value (613a, 613b, 613c, 613d) to a first lower pressure value (614a, 614b, 614c, 614d) with at most piecewise constant subintervals.

2. The anaesthesia- or ventilator apparatus according to claim 1, wherein the operating- and actuating unit (900) is further arranged to regulate the operation of the controllable expiratory valve (96) and of the ventilator drive (98) so that the decreasing profile from the first upper pressure value (613a, 613b, 613c, 613d) to the first lower pressure value (614a, 614b, 614c, 614d) has a linearly decreasing profile (601, 602, 604) and/or a non-linearly decreasing profile (603).

3. The anaesthesia- or ventilator apparatus according to claim 1, wherein the operating- and actuating unit (900) is further arranged to regulate the operation of the controllable expiratory valve (96) and of the ventilator drive (98) so that the decreasing profile from a first upper pressure value (613a, 613b, 613c, 613d) to a first lower pressure value (614a, 614b, 614c, 614d) has a continuously decreasing profile (601, 602, 603) and/or a profile (604) which comprises decreasing and constant subintervals.

4. The anaesthesia- or ventilator apparatus according to claim 1, wherein the operating- and actuating unit (900) is further arranged to regulate the operation of the controllable expiratory valve (96) and of the ventilator drive (98) so that the at least partially decreasing profile (108a, 108b) from a first upper pressure value (613a, 613b, 613c, 613d) to a first lower pressure value (614a, 614b, 614c, 614d) starts after a dwell time with a constant pressure level (107a, 107b).

5. The anaesthesia- or ventilator apparatus according to claim 1, wherein the operating- and actuating unit (900) is arranged to determine the first upper pressure value (613a, 613b, 613c, 613d) and the first lower pressure value (614a, 614b, 614c, 614d) as a function of settings of the ventilator- or anaesthesia apparatus.

6. The anaesthesia- or ventilator apparatus according to claim 5, wherein the operating- and actuating unit (900) is arranged to establish the first upper pressure value and the first lower pressure value and in so doing to use settings to PEEP or to the ventilator frequency and/or of the I/E ratio.

## Revendications

1. Appareil d'anesthésie ou de respiration avec commande d'une pression expiratoire finale à la fin d'une phase d'expiration (650) d'un patient, dans lequel l'appareil d'anesthésie ou de respiration présente un circuit respiratoire (50), dans lequel sont disposés un capteur de pression expiratoire (91), un capteur de pression inspiratoire (91), un détecteur de débit inspiratoire (93), un détecteur de débit expiratoire (95), une soupape expiratoire réglable (96) et un entraînement de respiration (98), et une unité de conduite et de commande (900), qui est raccordée aux composants précités dans le circuit respiratoire, **caractérisé en ce que** l'unité de conduite et de commande (900) est conçue ou réalisée pour réguler le fonctionnement de la soupape expiratoire réglable (96) et de l'entraînement de respiration (98) de telle manière que la courbe de pression (401a, 401b, 501a, 501b) pendant la phase d'expiration (650) depuis la fin de la phase d'inspiration (660) jusqu'au début de la prochaine phase d'inspiration suivante (670) présente une allure descendante (105a, 105b, 106a, 106b, 615a, 615b, 615c, 615d) depuis une première valeur de pression supérieure (613a, 613b, 613c, 613d) jusqu'à une première valeur de pression inférieure (614a, 614b, 614c, 614d) avec des intervalles partiels au maximum constants segment par segment.

2. Appareil d'anesthésie ou de respiration selon la revendication 1, dans lequel l'unité de conduite et de commande (900) est en outre conçue ou réalisée pour réguler le fonctionnement de la soupape expiratoire réglable (96) et de l'entraînement de respiration (98) de telle manière que l'allure descendante depuis la première valeur de pression supérieure (613a, 613b, 613c, 613d) jusqu'à la première valeur de pression inférieure (614a, 614b, 614c, 614d) présente une allure descendante linéaire (601, 602, 604) et/ou une allure descendante non linéaire (603).

3. Appareil d'anesthésie ou de respiration selon la revendication 1, dans lequel l'unité de conduite et de commande (900) est en outre conçue ou réalisée pour réguler le fonctionnement de la soupape expiratoire réglable (96) et de l'entraînement de respiration (98) de telle manière que l'allure descendante depuis une première valeur de pression supérieure (613a, 613b, 613c, 613d) jusqu'à une première valeur de pression inférieure (614a, 614b, 614c, 614d) présente une allure descendante de façon continue (601, 602, 603) et/ou une allure (604), qui comprend des intervalles partiels descendants et des intervalles partiels constants.

4. Appareil d'anesthésie ou de respiration selon la revendication 1, dans lequel l'unité de conduite et de commande (900) est en outre conçue ou réalisée pour réguler le fonctionnement de la soupape expiratoire réglable (96) et de l'entraînement de respiration (98) de telle manière que l'allure au moins partiellement descendante (108a, 108b) depuis une première valeur de pression supérieure (613a, 613b, 613c, 613d) jusqu'à une première valeur de pression inférieure (614a, 614b, 614c, 614d) commence après un temps de retard avec un niveau de pression constant (107a, 107b).

5. Appareil d'anesthésie ou de respiration selon la revendication 1, dans lequel l'unité de conduite et de commande (900) est conçue ou réalisée pour déterminer la première valeur de pression supérieure (613a, 613b, 613c, 613d) et la première valeur de pression inférieure (614a, 614b, 614c, 614d) en fonction de réglages de l'appareil de respiration ou d'anesthésie.

6. Appareil d'anesthésie ou de respiration selon la revendication 5, dans lequel l'unité de conduite et de commande (900) est conçue ou réalisée pour fixer la première valeur de pression supérieure et la première valeur de pression inférieure et pour utiliser des réglages pour la PEEP [Positive End Expiratory Pressure : Pression Expiratoire Finale Positive] ou la fréquence respiratoire et/ou le rapport I/E.
